# EUROPEAN PATENT APPLICATION

(11) **EP 4 535 002 A1**
(43) Date of publication of application: **09.04.2025**
(21) Application number: 23383008.2
(22) Date of filing: 02.10.2023
(51) Int. Cl.: G01N 33/569, A61K 39/02, C12N 1/20

(54) **A PROCESS FOR OBTAINING WHOLE CELL WALL ANTIGENS FROM A MICROORGANISM AND AN IMMUNOASSAY FOR DETECTING ANTIBODIES RELATED TO A MICROBIAL INFECTION USING SAID WHOLE CELL WALL ANTIGENS**

(71) Applicant: Divasa-Farmavic, S.A., 08503 Gurb (ES)
(72) Inventor: FERRÉ ALEMANY, JOSEP, Barcelona (ES); DEL SANTS CASTANY ROMA, MIQUEL, VIC (ES); CANO RIFÀ, ANNA, TORELLÓ (ES)
(74) Representative: Ponti & Partners, S.L.P

(57) **Abstract**

The present invention relates to a process for obtaining whole cell wall antigens from a microorganism. The present invention also relates to an immunoassay for detecting antibodies related to a microbial infection comprising the step of using the whole cell wall antigens. The present invention further relates to the use of the immunoassay in a method for analysing mammary glands health status, colostrum quality or milk tank quality.

## Description

### Field of the invention

The present invention relates to the field of immmunology. In particular the present invention relates to a process for obtaining whole cell wall antigens from a microorganism. The invention further relates to an immunoassay based on whole cell wall antigens from microorganisms in bodily fluids of an animal, for example milk, suitable to detect specific antibodies related to a microbial infections.

### Background

Detection of specific antibodies against microorganisms in bodily fluids have important limitations due to the lack of sensitivity and specificity and the presence of interfering antibodies.

Immunoassays based on specific proteins expressed by microorganisms as antigen relay on the expression of these proteins so it lacks sensitivity when these proteins are not expressed by a certain strain. Immunoassays based on crude extracts as antigen often lack specificity as several microbial proteins are widely expressed and even some proteins bind immunoglobulins (for instance *Staphylococcus aureus* protein A).

In addition, the presence of microbial antibodies is often of poor diagnostic value due to the presence of innate antibodies and linked antibodies to overcome infections that can interfere with the results.

Accordingly, there is a need to increase diagnostic value of immunoassays that detect microbial antibodies by increasing its sensitivity and specificity as well as reducing interferences.

The present invention overcome the above mentioned drawbacks by providing an immunoassay, e.g. immunochromatography, an ELISA and a Dot Blot, based on whole cell wall antigens from microorganisms as useful alternative to existing immunoassay methods.

### Summary of the invention

In a first aspect, the present invention relates to a process for obtaining whole cell wall antigens from a microorganism.

In a second aspect, the present invention relates to an immunoassay for detecting antibodies related to a microbial infection comprising the step of using the whole cell wall antigens obtained according to the first aspect.

In a third aspect, the present invention relates to the use of the immunoassay according to the second aspect in a method for analysing mammary glands health status, colostrum quality or milk tank quality. In other words, the present invention also relates to a method for analysing mammary glands health status, colostrum quality or milk tank quality comprising the step of using the immunoassay according to the second aspect.

### Detailed description of the invention

In a first aspect, the present invention relates to a process for obtaining whole cell wall antigens from a microorganism comprising the steps of:
a) growing a microorganism in a suitable medium;
b) washing and inactivating and/or denaturalizing and/or removing the external components of the cell wall of the microorganism, preferably cell wall surface proteins, obtained in step a) by at least one of the following treatments:
   - autoclaving;
   - strong acid and/or base treatment;
   - combined heat and detergent treatment;
   - organic solvent treatment;
   - chaotropic agent treatment;
   - enzymatic treatment;
   - reducing agent treatment.
c) optionally, washing the microorganism obtained in step b).
wherein the process does not include a step of lysing the microorganism.

In an embodiment, the present invention also relates to a process for obtaining whole cell wall antigens from a microorganism consisting of:
a) growing a microorganism in a suitable medium;
b) washing and inactivating and/or denaturalizing and/or removing the external components of the cell wall of the microorganism, preferably cell wall surface proteins, obtained in step a) by at least one of the following treatments:
   - autoclaving;
   - strong acid and/or base treatment;
   - combined heat and detergent treatment;
   - organic solvent treatment;
   - chaotropic agent treatment;
   - enzymatic treatment;
   - reducing agent treatment.
c) washing the microorganism obtained in step b), being this step optional.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It should be noted that the term "approximately" or "about" applied to the values used earlier and later in this document includes a margin of error of ± 5 %, such as, for example, ± 4 %, ± 3 %, ± 2 %, ± 1 %.

In the context of the present invention, the term "antigen" means a molecule or group of molecules (for instance a whole microorganism) having distinct surface features or epitopes capable of stimulating a specific immune response. Antibodies (immunoglobulins) are produced by the immune system in response to exposure to antigens.

In the context of the present invention, the term "whole cell wall antigen" means the components of any microorganism cell wall after being treated by a process disclosed in the present invention or any other equivalent process within the spirit and scope of the invention.

In the context of the present invention, the term "inactivating and/or denaturalizing and/or removing the external components of the cell wall of the microorganism, preferably cell wall surface proteins" means that any microorganism is subjected to a harsh environment known to significantly affect its components, structure and activity, preferably cell wall surface proteins, without breaking down cell wall structure. This inactivation and/or denaturalization and/or removing can be carried out by at least one of the following treatments:
- autoclaving;
- strong acid and/or base treatment;
- combined heat and detergent treatment;
- organic solvent treatment;
- chaotropic agent treatment;
- enzymatic treatment;
- reducing agent treatment.

In a preferred embodiment, said step b) is carried out by autoclaving the microorganism obtained in step a).

As mentioned above, there are different options to inactivate and/or denaturalize and/or remove the external components of the cell wall of the microorganism, preferably cell wall surface proteins, without significantly breaking down cell wall structure. These options are at least one of the following treatments:
- autoclaving;
- strong acid and/or base treatment;
- combined heat and detergent treatment;
- organic solvent treatment;
- chaotropic agent treatment;
- enzymatic treatment;
- reducing agent treatment.

In step a) microorganisms are usually grown from overnight to several days at 30-40°C in an suitable culture medium including, but not limited to, LB (Lysogeny Broth), BHI (Brain Heart Infusion), TSB (Tryptic Soy Broth) and Thioglycolate broth. Cells are thenwashed, for example, 3 to 5 times by centrifugation and kept in a specific treatment solution for 5 to 60 minutes in order to inactivate and/or denaturalize and/or remove the external components of the cell wall of the microorganism, preferably cell wall surface proteins, without breaking down cell wall structure. After this treatment cells are optionally washed 3 to 5 times by centrifugation before using them as antigen in an immunoassay.

The treatment solution can be kept at 4°C to 37°C or it can be heated up to 100°C.

In case of autoclaving it can be kept to pressurized saturated steam at 121 - 132 °C for 5-60 minutes at a pressure of 15 - 30 psi.

The treatment solution may contain one or several components able to inactivate and/or denaturalize and/or remove the external components of the cell wall of the microorganism, preferably cell wall surface proteins without breaking down cell wall structure. The following components are provided as examples:
- Surfactant at 0,1 to 10 wt% final concentration. By way of example, non ionic surfactant such as Tween 20 or Triton X-100, anionic surfactant such as SDS (Sodium dodecyl sulfate) and zwitterionic surfactant such as CHAPS can be employed.
- Acid and/or bases at 0,1 to 0,5 M final concentration. By way of example, acids such as HCl or acetic acid and/or bases such as NaOH or sodium bicarbonate can be employed.
- Reducing agents at 0,1 to 10 wt% final concentration. By way fo example, 2-mercaptoethanol or DTT (dithiothreitol) can be employed.
- Chaotropic agents at 1 to 8 M final concentration. By way of example, guanidinium chloride or urea can be employed.
- Organic solvents at 10 to 70 wt% final concentration. By way of example, alcohol, for example EtOH, or chloroform can be employed.
- Enzymes at 0,01 to 20 mg /ml final concentration. By way of example, lysozyme or proteinase K can be employed.

In another preferred embodiment, said microorganism of step a) is a gram-positive or gram-negative bacteria.

In a more preferred embodiment, said microorganism of step a) is a gram-positive bacteria selected from *S*. *aureus, S. uberis* and *S*. *dysgalactiae.*

In another more preferred embodiment, said microorganism of step a) is *Escherichia Coli.*

The procedure developed by the applicant eliminates most of the antigens from the cell wall (in fact, a significant decrease in the antibody recognition pattern is observed in **Tables 1-4** when whole cell wall antigens are evaluated in parallel with non-treated cell wall) but it surprisingly significantly improves assay diagnostic value because the remaining whole cell wall antigens have a better correlation with the infection. This procedure can be surprisingly applied to a wide range of different microorganisms.

Furthermore, based on said whole cell wall antigen the inventors have developed a rapid and effective immunoassay to obtain results of diagnostic value. In the context of the present invention, the term "diagnostic value" means that valuable information can be obtained from the results of a certain immunoassay. It includes, but are not limited to, infective situation, health status or sample specific value (for instance determination the colostrum protective value of a certain sample).

Accordingly, in a second aspect, the present invention relates to immunoassay for detecting antibodies related to a microbial infection, preferably a bacterial infection, comprising the step of using the whole cell wall antigens as obtained according to the first aspect of the invention, including any of the embodiments alone or in combination.

In the context of the present invention, the term "immunoassay" means any assay based on antigen-antibody binding. It includes, but are not limited to, ELISA, Western Blots and immunochromatography tests. In a preferred embodiment, said immunoassay according to the second aspect of the invention is selected from an enzyme-linked immunosorbent assay (ELISA), Immunochromatography and Dot Blot.

In the context of the present invention, the term "whole cell wall immunoassay" means any immunoassay that contains the whole cell wall antigen described in the present invention.

In the context of the present invention, the term "Immunochromatography" (also named Lateral Flow assay) means any immunoassay involving a chromatographic migration step. It can detect a single parameter or it can detect multiple parameters simultaneously.

In the context of the present invention, the term "whole cell wall immunochromatography" or analogous terms means any immunochromatography that contains the whole cell wall antigen as described in the present invention.

In the context of the present invention, the term "Dot Blot" means any immunoassay involving incubating the sample with a membrane blotted with an antigen of interest. It can detect a single parameter or it can detect multiple parameters simultaneously.

In the context of the present invention, the term "whole cell wall Dot Blot" or analogous terms means any Dot Blot that contains the whole cell wall antigen as described in the present invention.

In the context of the present invention, the term "ELISA" (Enzyme linked immunosorbent assay) means any immunoassay involving a signal recognition step involving an enzymatic reaction. It can detect a single parameter or it can detect multiple parameters simultaneously.

In the context of the present invention, the term "whole cell wall ELISA" or analogous terms means any ELISA that contains the whole cell wall antigen as described in the present invention.

In the context of the present invention, the term "infection" means the invasion of tissues by pathogens, their multiplication, and the reaction of host tissues to the infectious agent and the toxins they produce.

In the context of the present invention, "specific antibodies related to a microbial infection" means the detection of antibodies which presence correlates with a microbial infection or correlates with the protection of the animal against a certain microorganism infection. In a preferred embodiment, said microbial infection is a bacterial infection.

In another preferred embodiment, the antibodies related to a microbial infection are detected in a sample of an animal milk, preferably a mammal milk, even more preferably a cow milk.

In the context of the present invention, the term "milk of infected lactating cow" means bovine milk with a confirmed presence of a specific microbial infection.

In the context of the present invention, the term "milk of non-infected lactating cow" means bovine milk with a confirmed absence of a specific microbial infection.

In the context of the present invention, the term "lactating cow milk" means bovine milk obtained after the transition period and before the dry off period.

In the context of the present invention, the term "bovine colostrum" means bovine milk obtained just after calving.

In the context of the present invention, the term "cow milk tank" means the group of mixed bovine milks obtained after a milking process.

In a third aspect, the present invention relates to the use of the immunoassay according to the second aspect of the invention including any of the embodiments alone or in combination, for analysing mammary glands health status.

The present invention also relates to the use of the immunoassay according to the second aspect of the invention including any of the embodiments alone or in combination, in a method for analysing colostrum quality.

The present invention further relates to the use of the immunoassay according to the second aspect of the invention including any of the embodiments alone or in combination, in a method for analysing milk tank quality.

In other words, the present invention also relates to a method for analysing mammary glands health status, colostrum quality or milk tank quality comprising the step of using the immunoassay according to the second aspect of the invention.

A number of examples will be provided below that are intended to illustrate the invention and in no way limit the scope of the invention, which is established by the attached claims.

### Examples

### Example 1: Obtaining whole cell wall antigens.

Whole cell wall antigens can be obtained by the following procedure:
Option A: Microorganisms *(Staphylococcus aureus, Streptococcus uberis, Streptococcus agalactiae* or *Escherichia coli)* are growth overnight at 37°C in TSB medium. Cells are washed 3 times by centrifugation and autoclaved at 121°C during 20 minutes in a solution containing Tween at 0,1 %. Autoclaved cells are washed 3 to 5 times by centrifugation to remove debris and excess detergent before using them as antigen in an immunoassay.
Option B: Microorganisms (*Staphylococcus aureus, Streptococcus uberis, Streptococcus agalactiae* or *Escherichia coli*) are growth 28 days at 37°C in TSB medium. Cells are washed 3 times by centrifugation and autoclaved at 121°C during 20 minutes in a solution containing Tween at 0,1 %. Autoclaved cells are washed 3 to 5 times by centrifugation to remove debris and excess detergent before using them as antigen in an immunoassay.
Option C: Microorganisms (*Staphylococcus aureus, Streptococcus uberis, Streptococcus agalactiae* or *Escherichia coli*) are growth overnight at 37°C in TSB medium. Cells are washed 3 times by centrifugation and boiled at 100°C during 20 minutes in a solution containing Tween at 0,1 % and SDS at 1 %. Boiled cells are washed 3 to 5 times by centrifugation to remove debris and excess detergent before using them as antigen in an immunoassay.
Option D: Microorganisms (*Staphylococcus aureus, Streptococcus uberis, Streptococcus agalactiae* or *Escherichia coli*) are growth overnight at 37°C in TSB medium. Cells are washed 3 times by centrifugation and kept in a ethanol solution at 70% during 60 minutes. Cells are washed 3 to 5 times by centrifugation to remove debris and excess ethanol before using them as antigen in an immunoassay.

*Staphylococcus aureus, Streptococcus uberis, Streptococcus agalactiae, Escherichia coli* have been successfully used to obtain whole cell wall antigens but any other microorganism of interest can be used.

Non treated whole cell wall antigen (control antigen) can be obtained using the following procedure:
Control antigen: Microorganisms (*Staphylococcus aureus, Streptococcus uberis, Streptococcus agalactiae* or *Escherichia coli*) are growth overnight at 37°C in TSB medium. Cells are washed 3 times by centrifugation before using them as antigen in an immunoassay.

Comparative performance of each antigen was evaluated with immunochromatography (see tables 1-4 showing whole cell wall antigens performance compared with non-treated cell wall antigen (control antigen)).

**Table 1:**

| Milk sample | *Staphylococcus aureus* whole cell wall antigen | | | | Control antigen |
|---|---|---|---|---|---|
| | Option A | Option B | Option C | Option D | |
| Non infected *S.aureus* n°1 | - | - | - | - | + |
| Non infected *S.aureus* n°2 | - | - | - | - | - |
| Non infected *S.aureus* n°3 | - | - | - | - | - |
| Infected *S.aureus* n°1 | + | + | + | + | + |
| Infected *S.aureus* n°2 | + | + | + | + | + |
| Infected *S.aureus* n°3 | + | + | + | + | + |

**Table 2:**

| Milk sample | *Streptococcus uberis* whole cell wall antigen | | | | Control antigen |
|---|---|---|---|---|---|
| | Option A | Option B | Option C | Option D | |
| Non infected *S.uberis* n°1 | - | - | - | - | - |
| Non infected *S. uberis* n°2 | - | - | - | - | + |
| Non infected *S*. *uberis* n°3 | - | - | - | - | - |
| Infected *S*. *uberis* n°1 | + | + | + | + | + |
| Infected *S*. *uberis* n°2 | + | + | + | + | + |
| Infected *S*. *uberis* n°3 | + | + | + | + | + |

**Table 3:**

| Milk sample | *Streptococcus agalactiae* whole cell wall antigen | | | | Control antigen |
|---|---|---|---|---|---|
| | Option A | Option B | Option C | Option D | |
| Non infected *S.agalactiae* n°1 | - | - | - | - | - |
| Non infected *S.agalactiae* n°2 | - | - | - | - | + |
| Non infected *S.agalactiae* n°3 | - | - | - | - | - |
| Infected *S.agalactiae* n°1 | + | + | + | + | + |
| Infected *S.agalactiae* n°2 | + | + | + | + | + |
| Infected *S.agalactiae* n°3 | + | + | + | + | + |

**Table 4**

| Milk sample | *Escherichia coli* whole cell wall antigen | | | | Control antigen |
|---|---|---|---|---|---|
| | Option A | Option B | Option C | Option D | |
| Non infected *E.coli* n°1 | - | - | - | - | - |
| Non infected *E.coli* n°2 | - | - | - | - | - |
| Non infected *E.coli* n°3 | - | - | - | - | + |
| Infected *E.coli* n°1 | + | + | + | + | + |
| Infected *E.coli* n°2 | + | + | + | + | + |
| Infected *E.coli* n°3 | + | + | + | + | + |

### Example 2: Immunoassay using whole cell wall antigens.

The immunoassays are produced according to well described methods taking into account the specific characteristics of the whole cell wall antigens:
A: Whole cell wall antigens should be dispensed in the immunochromatography test line and Dot Blot membrane at a relative high concentration compared to typical protein antigens (1 microliter at OD600 of 10 each strip) and relatively large porous size nitrocellulose membranes should be used (for instance MDI CNPH membrane) to avoid nitrocellulose clogging by antigen.
B: Whole cell wall antigens should be coated in an ELISA plate using standard 0.1 M pH 9.6 carbonate buffer but it requires an overnight shaking step to ensure a correct coating procedure.

Immunochromatography, Dot Blot and ELISA assays can use protein G, protein A or antibodies against specific immunoglobulins (Anti IgG, Anti IgM, Anti IgA or Anti IgE) as detection system. Protein G and Anti IgA are the preferred detection systems.

Milks can be diluted from 1/10 to 1/10.000 using different immunossay buffers. A standard immunoassay buffer (PBS with Tween), a Tween / SDS buffer or an stringent buffer (Glycine, EDTA, NaCl, Tween and BSA) can be used to dilute samples.

### Example 3: Milk from lactating cows evaluated by whole cell wall immunochromatography, Dot Blot and ELISA.

120 microlitres of 1/1000 of a panel of diluted cow milks (infected lactating cows and non-infected lactating cows) were added in a whole cell wall immunochromatography assay with a colloidal gold protein G conjugate. If specific antibodies against the whole cell wall antigen were present test line appeared.

1000 microlitres of 1/1000 of a panel of diluted cow milks (infected lactating cows and non-infected lactating cows) were added in a Dot Blot membrane coated with whole cell wall antigen and incubated for 1 h in agitation. After washing, a colloidal gold protein G conjugate diluted in 1 ml of immunoassay buffer was added for 1 additional hour. If specific antibodies against the whole cell wall antigen were present a red blot appeared.

100 microlitres of 1/1000 of a panel of diluted cow milks (infected lactating cows and non-infected lactating cows) were added in a whole cell wall coated ELISA well. After 30 minutes incubation and a wash procedure a HRPO protein G conjugate was added. After an additional 30 minutes incubation a new wash was performed and TMB was added into the well. If specific antibodies against the whole cell wall antigen were present a blue colour appeared.

Results with diagnostic value were obtained using the 3 immunoassays (see tables 5-8 showing *Staphylococcus aureus, Streptococcus uberis, Streptococcus agalactiae* and *Escherichia coli* whole cell wall antigen immunochromatography, Dot Blot and ELISA results with infected lactating cow milk and non-infected lactating cow milk).

**Table 5**

| *Staphylococcus aureus* whole cell wall antigen | | | |
|---|---|---|---|
| Milk sample | Immunochromatography | Dot Blot | ELISA |
| Non infected *S.aureus* n°1 | - | - | - |
| Non infected *S.aureus* n°2 | - | - | - |
| Non infected *S.aureus* n°3 | - | - | - |
| Infected *S.aureus* n°1 | + | + | + |
| Infected *S.aureus* n°2 | + | + | + |
| Infected *S.aureus* n°3 | + | + | + |

**Table 6**

| *Streptococcus uberis* whole cell wall antigen | | | |
|---|---|---|---|
| Milk sample | Immunochromatography | Dot Blot | ELISA |
| Non infected *S.uberis* n°1 | - | - | - |
| Non infected *S. uberis* n°2 | - | - | - |
| Non infected *S. uberis* n°3 | - | - | - |
| Infected *S. uberis* n°1 | + | + | + |
| Infected *S. uberis* n°2 | + | + | + |
| Infected *S. uberis* n°3 | + | + | + |

**Table 7**

| *Streptococcus agalactiae* whole cell wall antigen | | | |
|---|---|---|---|
| Milk sample | Immunochromatography | Dot Blot | ELISA |
| Non infected *S.agalactiae* n°1 | - | - | - |
| Non infected *S.agalaciae* n°2 | - | - | - |
| Non infected *S.agalactiae* n°3 | - | - | - |
| Infected *S.agalactiae* n°1 | + | + | + |
| Infected *S.agalactiae* n°2 | + | + | + |
| Infected *S.agalactiae* n°3 | + | + | + |

**Table 8**

| *Escherichia coli* whole cell wall antigen | | | |
|---|---|---|---|
| Milk sample | Immunochromatography | Dot Blot | ELISA |
| Non infected *E.coli* n°1 | - | - | - |
| Non infected *E.coli* n°2 | - | - | - |
| Non infected *E.coli* n°3 | - | - | - |
| Infected *E*.*coli* n°1 | + | + | + |
| Infected *E.coli* n°2 | + | + | + |
| Infected *E*.*coli* n°3 | + | + | + |

### Example 4: Bovine colostrum evaluated by whole cell wall immunochromatography, Dot Blot and ELISA.

120 microlitres of 1/1000 of a panel of diluted bovine colostrums were assayed with a whole cell wall immunochromatography assay with a colloidal gold protein G conjugate. If specific antibodies against the whole cell wall antigen were present test line appears.

1000 microlitres of 1/1000 of a panel of diluted bovine colostrums were assayed with a Dot Blot membrane coated with whole cell wall antigen and incubated for 1 h under agitation. After washing, a colloidal gold protein G conjugate diluted in 1 ml of immunoassay buffer was added for 1 additional hour. If specific antibodies against the whole cell wall antigen were present a red blot appears.

100 microlitres of 1/1000 of a panel of diluted bovine colostrums were assayed with a whole cell wall coated ELISA well. After 30 minutes incubation and a wash procedure a HRPO protein G conjugate was added. After an additional 30 minutes incubation a new wash was performed and TMB was added into the well. If specific antibodies against the whole cell wall antigen were present a blue colour appears.

Results with diagnostic value were obtained using the 3 immunoassays (see tables 9-11 showing *Staphylococcus aureus, Streptococcus uberis, Streptococcus agalactiae* and *Escherichia coli* whole cell wall antigen immunochromatography, Dot Blot and ELISA results with bovine colostrum).

**Table 9**

| Bovine colostrum sample | Immunochromatography whole cell wall antigen | | | |
|---|---|---|---|---|
| | *Staphylococcus aureus* | *Streptococcus uberis* | *Streptococcus agalactiae* | *Escherichia coli* |
| Sample n°1 | + | + | - | - |
| Sample n°2 | - | + | - | - |
| Sample n°3 | - | + | - | - |
| Sample n°4 | + | + | + | + |

**Table 10**

| Bovine colostrum sample | Dot Blot whole cell wall antigen | | | |
|---|---|---|---|---|
| | *Staphylococcus aureus* | *Streptococcus uberis* | *Streptococcus agalactiae* | *Escherichia coli* |
| Sample n°1 | + | + | - | - |
| Sample n°2 | - | + | - | - |
| Sample n°3 | - | + | - | - |
| Sample n°4 | + | + | + | + |

**Table 11**

| Bovine colostrum sample | ELISA whole cell wall antigen | | | |
|---|---|---|---|---|
| | *Staphylococcus aureus* | *Streptococcus uberis* | *Streptococcus agalactiae* | *Escherichia coli* |
| Sample n°1 | + | + | - | - |
| Sample n°2 | - | + | - | - |
| Sample n°3 | - | + | - | - |
| Sample n°4 | + | + | + | + |

### Example 5: Cow milk tank evaluated by whole cell wall immunochromatography, Dot Blot and ELISA

120 microlitres of 1/100 of a panel of diluted cow milk tank samples were added in a cell wall immunochromatography assay with a colloidal gold protein G conjugate. If specific antibodies against the whole cell wall antigen were present test line appears.

1000 microlitres of 1/100 of a panel of diluted cow milk tank samples were added in a Dot Blot membrane coated with cell wall antigen and incubated for 1 h under agitation. After washing, a colloidal gold protein G conjugate diluted in 1 ml of immunoassay buffer was added for one additional hour. If specific antibodies against the whole cell wall antigen were present a red blot appears.

100 microlitres of 1/100 of a panel of diluted cow milk tank samples were added in a cell wall coated ELISA well. After 30 minutes incubation and a wash procedure a HRPO protein G conjugate was added. After an additional 30 minutes incubation a new wash was performed and TMB was added into the well. If specific antibodies against the whole cell wall antigen were present a blue colour appears.

Results with diagnostic value were obtained using the 3 immunoassays (see tables 12-14 showing *Staphylococcus aureus, Streptococcus uberis, Streptococcus agalactiae* and *Escherichia coli* whole cell wall antigen immunochromatography, Dot Blot and ELISA results with cow tank milk).

**Table 12:**

| Cow tank milk sample | Immunochromatography whole cell wall antigen | | | |
|---|---|---|---|---|
| | *Staphylococcus aureus* | *Streptococcus uberis* | *Streptococcus agalactiae* | *Escherichia coli* |
| Sample n°1 | - | - | - | - |
| Sample n°2 | + | - | - | - |
| Sample n°3 | - | + | - | - |

**Table 13:**

| Cow tank milk sample | Dot Blot whole cell wall antigen | | | |
|---|---|---|---|---|
| | *Staphylococcus aureus* | *Streptococcus uberis* | *Streptococcus agalactiae* | *Escherichia coli* |
| Sample n°1 | - | - | - | - |
| Sample n°2 | + | - | - | - |
| Sample n°3 | - | + | - | - |

**Table 14:**

| Cow tank milk sample | ELISA whole cell wall antigen | | | |
|---|---|---|---|---|
| | *Staphylococcus aureus* | *Streptococcus uberis* | *Streptococcus agalactiae* | *Escherichia coli* |
| Sample n°1 | - | - | - | - |
| Sample n°2 | + | - | - | - |
| Sample n°3 | - | + | - | - |

### Discussion of examples and conclusion

1: Unsatisfactory results developing an immunochromatography, a Dot Blot and an ELISA following procedures described in the literature (Reference 1 and Reference 2) were obtained by the applicant. A weak correlation between milk of infected and non-infected lactating cows were obtained due to the presence of large number of false positive and negative results. Alternative approaches were explored by the applicant in order to try to improve assay diagnostic value.

Surprisingly, a more specific and sensitive result was obtained when whole cell wall antigens as obtained according to **Example 1** were evaluated.

The whole cell wall antigen production procedures developed by the applicant inactivates and/or denaturalizes and/or removes most of the cell wall surface proteins as well as other external components of the cell wall without breaking down the cell wall. This procedure can be achieved by incubating microorganism cells in a harsh environment like autoclaving, strong acid and/or base treatment and combined heat and detergent treatment.

The procedure developed by the applicant eliminates most of the antigens from the cell wall (in fact, a significant decrease in the antibody recognition pattern is observed in **tables 1-4** when whole cell wall antigens are evaluated in parallel with non-treated cell wall) but it surprisingly significantly improves assay diagnostic value because the remaining whole cell wall antigens have a better correlation with the infection. This procedure can be surprisingly applied to a wide range of different microorganisms.

Specificity of whole cell wall antigens were checked using milk samples with very satisfactory results. Surprisingly, cow milks with *Streptococcus uberis* infection have antibodies that bind to *Streptococcus uberis* whole cell wall antigen but do not have antibodies that bind to *Staphylococcus aureus* whole cell wall antigen and vice versa (**Tables 5-8**). In addition, binding profile of different colostrums is very variable using different whole cell wall antigens (**Tables 9-11**). These results suggest only specific antibodies are bound to each whole cell wall antigen.

It is described in the literature that cell wall composition can show notable differences within the same microbial specie (Reference 3). Cell wall antigens obtained using different culture conditions (18 h culture versus 28 days culture) provide equivalent results when milk of infected and non-infected lactating cows from different sources were assayed (**Tables 1-4**) so whole cell wall antigens are antigens with a surprisingly good uniformity.

*Staphylococcus aureus, Streptococcus uberis, Streptococcus agalactiae* and *Escherichia coli* have ben successfully used to obtain whole cell wall antigens. Any other microorganism of interest can be used.

Most of the described antigen production processes used to detect specific microbial antibodies relay on the use of specific recombinant or purified proteins as antigens. Published whole cell antigens used to detect microbial antibodies are usually sonicated extracts where cell wall is disrupted and most of the proteins are not significantly modified (Reference 5).

2: A whole cell wall immunochromatography, a Dot Blot and whole cell wall ELISA were developed using protocols described in the literature (Reference 1 and Reference 2) but no satisfactory results were obtained (a very poor signal was obtained). In order to overcome this problem different approaches were explored by the applicant.

Due to the specific characteristics of the whole cell wall antigen some modifications in the procedure described in the literature were incorporated as described in **Example 2.**

Surprisingly a very strong positive signal was obtained with the whole cell wall immunochromatography and Dot Blot assay when a high concentration of whole cell wall antigen combined with a relatively large porous nitrocellulose membrane was used. In addition, also a surprisingly good uniform signal was obtained with ELISA when whole cell wall antigens were coated under agitation overnight.

A significant proportion of milk samples from uninfected cows tested positive when analyzed with an immunoassay containing the antigens of the present invention together with buffers and sample dilutions described in the literature (references 2 and 4). In order to improve the diagnostic value of the antigen of the present invention different tests were carried out.

Surprisingly when the assay was performed using a high concentration of antigen combined with a marked dilution of the sample (1/10 - 1/10,000) and protein G as conjugate (see example 2) an improvement in the diagnostic value was significantly observed.

In other words, the antigen of the present invention allows for the correct detection of most milks evaluated when using, for example, a high concentration of antigen and a marked sample dilution (1/10-1/10,000) and protein G as conjugate (see example 2). The possibility of using high dilutions of the sample significantly limits possible interferences due to variations in the composition of the tested milk samples.

It should be understood, however, that the detailed description and the specific example, while indicating preferred embodiments of the invention, is given by way of illustration only, since various changes and modifications of the immunoassay buffer and the detection system (for instance the use of an anti IgA instead of protein G) and the format of the immunoassay within the spirit and scope of the invention will become apparent to those skilled in the art from this detailed description.

Unexpectedly, milk of infected lactating cows contains specific antibodies that seem to bind to whole cell wall antigen significantly better than milk of non-infected lactating cows under these assay conditions.

**3:** Finally, in order to confirm the diagnostic value of the whole cell wall immunoassays they were evaluated with cow milk from different sources by the applicant.

Whole cell wall antigen developed by the applicant significantly increases diagnostic value of the immunoassays. Whole cell wall antigens can be easily mixed (for instance it allows to detect different infections simultaneously) and a wide range of different antigens are readily available (for instance, it allows lactating milk, colostrum and tank milk screening for the presence of a wide range of microorganisms). In addition, as high dilutions of cow milk can be evaluated it is possible to easily evaluate pooled milk samples by decreasing the dilution (for instance it allows milk tank analysis at different dilutions).

Whole cell wall immunoassays surprisingly allowed to correctly classify milks of infected lactating cows and milks of non-infected lactating cows (**Example 3 and Tables 5-8**). An immunoassay based on whole cell wall antigen can be used to analyse mammary glands health status by detecting the presence of microbial infections (presence of specific antibodies correlates with the presence of an infection in samples evaluated in **Example 3 and Tables 5-8**).

Whole cell wall immunoassays surprisingly allowed to differentiate bovine colostrum samples (**Example 4 and Tables 9-11**). An immunoassay based on whole cell wall antigen can be used to analyse colostrum quality by detecting the presence of different antimicrobial antibodies (different passive immunity transfer to calves is expected from the different samples evaluated in **Example 4 and Tables 9-11**).

Finally, whole cell wall immunoassays surprisingly allowed to analyse cow milk tank quality of samples from different sources (**Example 5 and Tables 12-14**). An immunoassay based on whole cell wall antigen can be used to analyse milk tank quality by detecting the presence of different antimicrobial antibodies linked to infections (*Staphylococcus aureus* infection is suspected to be present in milk tank sample 2 and *Streptococcus uberis* infection is suspected to be present in milk tank sample 3 in **Example 5 and Tables 12-14**).

It should be understood, however, that detailed examples of the diagnostic value of the develop cell wall immunoassays as shown above, while indicating preferred embodiments of the invention, is given by way of illustration only, since any other use with diagnostic value of the developed whole cell wall immunoassays within the spirit and scope of the invention will become apparent to those skilled in the art from this disclosure.

### REFERENCES

**Reference 1:** Pernthaner, A (2019) Device for detection of antibodies to a pathogen WO2019216775A1
**Reference 2:** Systemic and local immune response of cows to intramammary infection with Staphylococcus aureus. Leitner G, Yadlin B, Glickman A, Chaffer M, Saran A. Res Vet Sci. 2000 Oct;69(2):181-4. doi: 10.1053/rvsc.2000.0409. PMID: 11020372
**Reference 3:** Molecular mapping of the cell wall polysaccharides of the human pathogen Streptococcus agalactiae. Beaussart A, Péchoux C, Trieu-Cuot P, Hols P, Mistou MY, Dufrêne YF. Nanoscale. 2014 Dec 21;6(24):14820-7. doi: 10.1039/c4nr05280c. Epub 2014 Oct 31. PMID: 25358409
**Reference 4:** Wondu W-M (2002) A method for simultaneous detection of multiple microbial antigens in biological specimens from mastitic animals. Patent application number WO 2002075310 A
**Reference 5:** Development of a Gold Nanoparticle Based Lateral Flow Assay for Rapid Diagnosis of Contagious Agalactia in Goats T.R. Arun, R. Rana, P. Singh, P. Choudhuri, V.P. Singh, P. Thomas, V. Rekha, K. Nehra, J. Usharani and K. Dhama. Asian Journal of Animal and Veterinary Advances Year: 2014 | Volume: 9 | Issue: 7 | Page No.: 405-413 DOI: 10.3923/ajava.2014.405.413

## Claims

1. A process for obtaining whole cell wall antigens from a microorganism comprising the steps of:
a) growing a microorganism in a suitable medium;
b) washing and inactivating and/or denaturalizing and/or removing the external components of the cell wall of the microorganism, preferably cell wall surface proteins, obtained in step a) by at least one of the following treatments:
- autoclaving;
- strong acid and/or base treatment
- combined heat and detergent treatment;
- organic solvent treatment;
- chaotropic agent treatment;
- enzymatic treatment;
- reducing agent treatment.
c) optionally, washing the microorganism obtained in step b).
wherein the process does not include a step of lysing the microorganism.

2. The process according to claim 1, wherein said step b) is carried out by autoclaving the microorganism obtained in step a).

3. The process according to claim 1 or 2, wherein said microorganism is a gram-positive or gram-negative bacteria.

4. The process according to claim 3, wherein said microorganism is a gram-positive bacteria selected from *S*. *aureus, S. uberis* and *S*. *dysgalactiae.*

5. Process according to claim 3, wherein said microorganism is *Escherichia Coli.*

6. An immunoassay for detecting antibodies related to a microbial infection comprising the step of using the whole cell wall antigens obtained according to any of claims 1 to 4.

7. The immunoassay according to claim 6, wherein said microbial infection is a bacterial infection.

8. The immunoassay according to claim 6 or 7, wherein said immunoassay is selected from an enzyme-linked immunosorbent assay (ELISA), Immunochromatography and Dot Blot.

9. The immunoassay according to any of claims 6 to 8, wherein the antibodies are detected in a sample of an animal milk.

10. The immunoassay according to claim 9, wherein said animal milk is a mammal milk.

11. The immunoassay according to claim 10, wherein said mammal milk is a cow milk.

12. Use of the immunoassay according to any of claims 9 to 11 in a method for analysing mammary glands health status.

13. Use of the immunoassay according to any of claims 9 to 11 in a method for analysing colostrum quality.

14. Use of the immunoassay according to any of claims 9 to 11 in a method for analysing milk tank quality.
